# EUROPEAN PATENT SPECIFICATION

(11) **EP 3 726 995 B1**
(45) Date of publication and mention of the grant of the patent: **29.07.2026**
(21) Application number: 18815767.1
(22) Date of filing: 17.12.2018
(51) Int. Cl.: A01N 65/03, A01P 21/00

(54) **METHOD OF ISOLATING AND PURIFYING BIOACTIVE COMPOUNDS FROM SEAWEED EXTRACTS, EXTRACT AND METHOD OF USE**
VERFAHREN ZUR ISOLIERUNG UND REINIGUNG VON BIOAKTIVEN VERBINDUNGEN AUS SEETANGEXTRAKTEN, EXTRAKT UND VERFAHREN ZUR VERWENDUNG
PROCÉDÉ D'ISOLEMENT ET DE PURIFICATION DE COMPOSÉS BIOACTIFS À PARTIR D'EXTRAITS D'ALGUES, EXTRAIT ET MÉTHODE D'UTILISATION

(30) Priority: 18.12.2017 FR 1762345
(43) Date of publication of application: 28.10.2020
(73) Proprietor: Laboratoires Goëmar, 35435 Saint Malo (FR)
(72) Inventor: CONAN, Céline, 35350 SAINT COULOMB (FR); POTIN, Philippe, 29680 ROSCOFF (FR); GUIBOILEAU, Anne, 35350 SAINT-MELOIR DES ONDES (FR); BESSE, Samantha, 64420 ESLOURENTIES DABAN (FR); JOUBERT, Jean-Marie, 35400 SAINT MALO (FR)
(74) Representative: J A Kemp LLP
(86) International application number: PCT/EP2018/085254
(87) International publication number: WO 2019/121539

(56) References cited:
- MARCELO CATARINO ET AL: "Fucaceae: A Source of Bioactive Phlorotannins", INTERNATIONAL JOURNAL OF MOLECULAR SCIENCES, vol. 18, no. 6, 21 June 2017 (2017-06-21), pages 1327, XP055499206, DOI: 10.3390/ijms18061327
- KATARZYNA CHOJNACKA ET AL: "Biologically Active Compounds in Seaweed Extracts - the Prospects for the Application", THE OPEN CONFERENCE PROCEEDINGS JOURNAL, vol. 3, no. 1, 6 August 2012 (2012-08-06), pages 20 - 28, XP055132387, ISSN: 2210-2892, DOI: 10.2174/1876326X01203020020

## Description

### FIELD OF THE INVENTION

The present invention relates generally to a method of purifying and isolating bioactive compounds responsible for plant growth stimulation from seaweed extracts.

### BACKGROUND OF THE INVENTION

One of the challenges of modern agriculture is to address the societal demand for sustainability, quality, and safety in agricultural production and to adapt itself to the world population increase by improving yields and crop tolerance to a changing environment.

Biostimulants can be used to improve plant nutrition, which impacts yield and quality parameters. Plant biostimulants generally fall within one of these categories i.e. hormone-containing products, plant extract based products, micronutrients based products, amino acid-containing products and humic acid-containing products but may not be strictly restricted to these categories alone. Plant biostimulants are used to treat crops in a commercial setting in view of their ability to increase growth rates, increase stress tolerance, increase photosynthetic rate and increase disease tolerance. Plant biostimulants are generally believed to operate by up-regulating or down-regulating key biological pathway genes.

As defined by the European Biostimulant Industry Council (EBIC), plant biostimulants contain substance(s) and/or micro-organisms whose function when applied to plants or the rhizosphere is to stimulate natural processes to enhance/benefit nutrient uptake, nutrient efficiency, tolerance to abiotic stress, and crop quality. Biostimulants have no direct action against pests, and therefore do not fall within the regulatory framework of pesticides.

Biostimulants are available in a variety of formulations and with varying ingredients but are generally classified on the basis of their source and content. These groups include humic substances (HS), and amino acid containing products (AACP).

Biostimulants are available in a variety of formulations and with varying ingredients but are generally classified into seven main groups on the basis of their source and content. These groups include humic substances (humic and fluvic acids), protein hydrolysates and other N-containing compounds, seaweed extracts and botanicals, chitosan and other biopolymers, inorganic compounds, beneficial fungi and beneficial bacteria.

Despite the commercially availability of numerous fertilizers and plant biostimulants, there continues to be a demand for improved products capable of serving a variety of needs. Therefore, new products and methods for improving plant growth responses and development are needed.

Seaweed and seaweed-derived products have been widely used in crop production systems due to the presence of a number of plant growth-stimulating compounds within these products. Thus, the biostimulation potential of many of these products has not been fully exploited due to the lack of scientific data on growth factors present in seaweeds and their various modes of action in affecting plant growth.

While the physiological effects of seaweed extracts on plant defenses and plant growth has been examined, little is known about the particular bioactive compounds in seaweed extracts that are responsible for these plant stimulants. It would be desirable to accelerate the identification of these bioactive components in algae, including, for example, brown algae extracts.

Phaeophyceae or brown algae are a large group of mostly marine multicellular algae, including many types of seaweed located in both Hemisphere waters. They play an important role in marine environments, both as food and as habitats. Many brown algae, such as members of the order Fucales, commonly grow along rocky seashores. Worldwide, over 1,500 species of brown algae are known. Some species, such as *Ascophyllum nodosum,* are important in commercial use and have environmental impact as well.

U.S. Pat. No. 7,611,716 to Michailovna et al describes a method of processing seaweed to obtain, in a single process, extracts comprising acidic and neutral polysaccharides and an extract comprising low molecular weight biologically active compounds that can be used in medicine, food, perfumery and the cosmetic industry. However, the reference only describes a method of processing seaweed and does not provide any way of identifying potential plant biostimulant compounds contained therein.

U.S. Pat. No. 3,856,569 to Strong, describes a method of purifying and concentration of the desirable polyssacharide such as carrageenan or alginate from aqueous solutions derived from marine algae (Rhodophyceae and Phaeophyceae) by subjecting the solutions to ultrafiltration. However, again, this reference only provides a method of processing seaweed and does not provide any way of identifying biostimulant compounds contained therein.

Catarino et al. (International Journal of Molecular Sciences, vol. 18, no. 6, 21 June 2017, page 1327) describes the therapeutic, and other industrially useful, properties of *Fucaceae* macroalgae. Chojnacka et al. (The Open Conference Proceedings Journal, vol. 3, no. 1, 6 August 2012, pages 20 to 28) describes utilitarian properties of algal extracts, including their application as components of pharmaceuticals, feeds for animals and fertilizers.

Because of the growing demand on products that are organic, environmentally friendly and harmless to human health, the need for natural biostimulants has increased. In addition, there is a need for similar or more effective biostimulants than the traditional biostimulants that have been used. In addition, there remains a need in the art for an improved process of isolating and purifying biostimulant compounds, including from extracts derived from seaweed.

### OBJECTS OF THE INVENTION

It is an object of the present invention to identify and purify substances that can be used as plant biostimulants.

It is another object of the present invention to identify and purify bioactive compounds in various seaweed extracts that are responsible for plant growth stimulation.

### SUMMARY OF THE INVENTION

The invention is as defined in the claims.

The present invention provides a fraction isolated from an algal species, comprising one or more bioactive molecules having a molecular weight in the range of 0.15 kDa to 1.0 kDa, wherein:
the fraction is obtainable by ultrafiltration of an extract of the algal species using a 1 kDa MWCO membrane, and the one or more bioactive molecules do not comprise a sulfated polysaccharide or laminarin;
the algal species is a brown algal species selected from the group comprising *Ascophyllum nodosum, Fucus vesiculosus, Sargassum* sp., and combinations thereof; and
the fraction is for improving plant growth.

The present invention also provides a method of improving plant growth, the method comprising the step of applying a composition comprising a fraction according to the present invention to at least one of soil, a plant, or a seed.

The present invention also provides a method of isolating and purifying bioactive compounds in an extract obtained from seaweed, the method comprising the steps of:
a) circulating the extract through an ultrafiltration membrane having a suitable molecular weight cutoff of 1 kDa or less;
b) collecting filtrate from the extract to obtain a first filtrate fraction and a retentate; and
c) rinsing the retentate to obtain one or more additional filtrate fractions.

The present invention also provides use of a fraction isolated from an algal species to improve plant growth, the fraction comprising one or more bioactive molecules having a molecular weight in the range of 0.15 kDa to 1.0 kDa, wherein:
the fraction is obtainable by ultrafiltration of an extract of the algal species using a 1 kDa MWCO membrane, and the one or more bioactive molecules do not comprise a sulfated polysaccharide or laminarin; and
the algal species is a brown algal species selected from the group comprising *Ascophyllum nodosum, Fucus vesiculosus, Sargassum* sp., and combinations thereof.

### GENERAL DISCLOSURE

Disclosed herein are one or more bioactive molecules isolated from an algae species, the one or more bioactive molecules having a molecular weight in the range of about 0.15 kDa to about 1.0 kDa.

Also described herein is a method of isolating and purifying bioactive compounds in an extract obtained from seaweed, the method comprising the steps of:
a) circulating the extract through an ultrafiltration membrane having a suitable molecular weight cutoff;
b) collecting filtrate from the seaweed extract to obtain a first filtrate fraction and a retentate; and
c) rinsing the retentate to obtain one or more additional filtrate fractions.

### BRIEF DESCRIPTION OF THE FIGURES

For a fuller understanding of the invention, reference is made to the following description taken in connection with the accompanying figures, in which:
Figures 1a and 1b depict Size Exclusion Chromatography (SEC) fractionation performed on filtered RM-3496 extract and a chromatogram of standards injected on the SEC to evaluate the average molecular weights of the molecules presented in the different fractions.
Figure 2 depicts boxplots showing the efficacy of SEC fractionation of RM-3496 on lettuces.
Figures 3a and 3b depict boxplots showing the fresh shoot weights and the fresh root weights of *in-vitro* cultures of *Arabidopsis thaliana* treated with the F3 fraction as compared with the untreated control, the RM-3496 extract and the rebuilt RM-3496 extract.
Figure 4 depicts a view of the ultrafiltration process in accordance with one aspect of the present invention.
Figure 5 depicts boxplots showing the fresh shoot weights of lettuces treated with various ultrafiltrated fractions, retentates and extracts.
Figure 6 depicts boxplots showing the fresh shoot weights of wheats treated with various ultrafiltrated fractions, retentates and extracts.

### DETAILED DESCRIPTION OF THE PREFERRED EMBODIMENTS

By plant "biostimulant" what is meant is an organic material that contains substance(s) and/or micro-organisms whose function when applied to plants or the rhizosphere is to stimulate natural processes to enhance/benefit nutrient uptake, nutrient efficiency, tolerance to abiotic stress, and crop quality.

When introducing elements of the present invention or the preferred embodiments(s) thereof, the articles "a", "an", "the" and "said" are intended to mean that there are one or more of the elements. The terms "comprising", "including" and "having" are intended to be inclusive and mean that there may be additional elements other than the listed elements.

As used herein, the term " about" refers to a measurable value such as a parameter, an amount, a temporal duration, and the like and is meant to include variations of +/-15% or less, preferably variations of +/-10% or less, more preferably variations of +/-5% or less, even more preferably variations of +/-1% or less, and still more preferably variations of +/-0.1% or less of and from the particularly recited value, in so far as such variations are appropriate to perform in the invention described herein. Furthermore, it is also to be understood that the value to which the modifier "about" refers is itself specifically disclosed herein.

The present invention describes a method of purifying and isolating biostimulant compounds from extracts derived from seaweed that are capable of increasing growth rates and yields of a wide range of crops.

The present invention provides a method of purifying the bioactive compounds responsible for plant growth stimulation in seaweed extracts by the metabolomics profiling of the seaweed extracts.

The fraction of the present invention comprises one or more bioactive molecules isolated from an algal species, the one or more bioactive molecules having a molecular weight in the range of 0.15 kDa to 1.0 kDa. The one or more bioactive molecules are ones that are capable of improving plant growth. The algal species is a brown algal species selected from the group comprising *Ascophyllum nodosum, Fucus vesiculosus, Sargassum* sp., and combinations of one or more of the foregoing. The one or more bioactive molecules do not comprise a sulfated polysaccharide or laminarin.

The present invention also provides a method of improving plant growth, the method comprising the step of applying a composition comprising the fraction according to the present invention. Improving plant growth may include at least one of the following: promoting seed germination, stimulating root development, prolonging a vegetative period, increasing a period of production, or increasing a period of harvest.

The present invention also provides a method of isolating and purifying bioactive compounds in an extract obtained from seaweed, the method comprising the steps of:
a) circulating the extract through an ultrafiltration membrane having a suitable molecular weight cutoff of 1 kDa or less;
b) collecting filtrate from the extract to obtain a first filtrate fraction and a retentate; and
c) rinsing the retentate to obtain one or more additional filtrate fractions.

The method may further comprise the step of evaluating the bioactivity of the first filtrate fraction and the additional filtrate fractions to determine their efficacy on plant growth. The efficacy on plant growth may include at least one of the following: promoting seed germination, stimulating root development, prolonging a vegetative period, increasing a period of production, or increasing a period of harvest. The extract may be produced from a brown algal species. The extract may be obtained from Ascophyllum nodosum, Fucus vesiculosus, or Sargassum sp. algae.

The retentate may comprise active molecules selected from the group consisting of sulfated polysaccharides and laminarin, which are active molecules capable of alleviating abiotic stress, such as salt excess, in crops. The first filtrate may comprise bioactive molecules having a molecular weight in the range of about 0.15 kDa to about 1.0 kDa.

The ultrafiltration membrane used in the method of isolating and purifying according to the invention has a molecular weight cutoff (MWCO) of 1 kDa or less.

*Ascophyllum nodosum* (rockweed) is a brown algal fucoid species found in the North Atlantic Ocean and has been used as a source of biostimulant for agricultural crops in order to improve plant growth, plant productivity and food quality.

Several purification techniques were assayed to desalt and purify these seaweed extracts according to the polarity and the size of molecules. For each purification procedure, the fractions were assayed on lettuces to ensure that the bioactivity was kept in the different desalted fraction or remained associated with the salts. This purification step was applied on a seaweed extract (RM-2705, a fucoid extract available from Goëmar) and the non-polar purified fractions showed a high purity level. The majority of the biomolecules (about 69%) were co-eluted with salts in order to show that the bioactive molecules were located in one of the non-polar purified fractions. Thereafter, the different fractions were tested on lettuce in comparison with the whole extract. However, the results showed that the bioactive molecules responsible for plant growth stimulation were present in the polar fraction that also contained salts. It was determined that fractionation according to the polarity of the molecules contained in RM-2705 and RM-3496 extracts was not suitable for desalting and purifying the seaweed extracts. Indeed, for all fractionation techniques used, the bioactive molecules remained associated with salts.

Thus, the challenge arose to find another method of desalting the seaweed extracts, while maintaining the plant growth biostimulant activity. Various purification techniques were investigated, including Liquid Liquid Extraction (LLE) with ethyl acetate, Solid Phase Extraction (SPE) with different sorbents (a normal phase: cyanopropyl-silica and a reverse phase" Amberlite^{®} XAD2), Solid Liquid Extraction (SLE) with butanol and Size Exclusion Chromatography (SEC).

In addition, in order to obtain information regarding the stability of the bioactive molecules, the heat stability of the activity of the RM-2705 extract was assayed on lettuces. The results showed a heat stability of the bioactive molecules, and autoclaving or boiling of the treated extracts enhanced the free shoot weights of lettuces.

The fractionation according to the polarity appeared to be inefficient in purifying the bioactive molecules, so the seaweed extract was fractionated according to the particle size of its molecules to attempt its desalting.

It was determined that all of these techniques appeared inefficient for the purpose except for Size Exclusion Chromatography (SEC) with Superdex^{®}30 resin, also referred to as Gel Filtration Chromatography (GF). SEC was found to be the only effective method for desalting and purifying the seaweed filtrate.

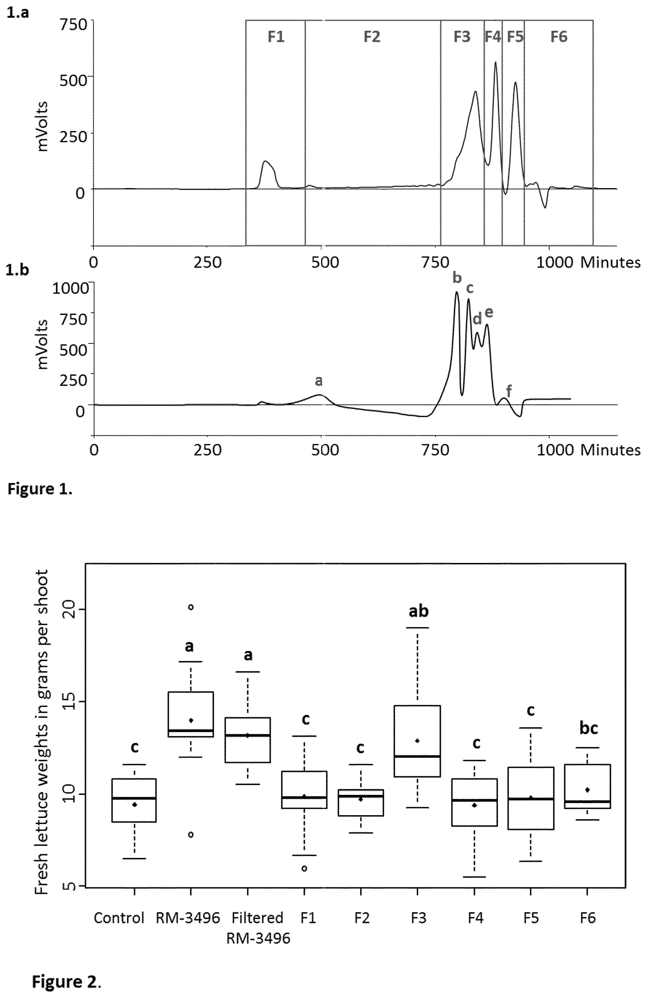

Based thereon, the seaweed extract (RM-3496) was fractionated by the SEC fractionation process and the molecules were eluted according to their size (or molecular weights) as shown in Figure 1.a. ASuperdex30^{®} resin (GE Healthcare, Bjorkgatan, Sweden) was used to ensure a good separation of molecules with a molecular weight below 10 kDa. The smaller the molecules (i.e., lower molecular weights), the more they are trapped in the porous beads of the gel and are eluted later. Thus, the molecular weights of the molecules decrease from the first fraction (i.e., F1) to the last fraction (i.e., F6). The fractions F1 and F2 were constituted by the larger molecules that flow through the column faster than the salts and the very low molecular weight molecules are eluted in fractions F5 and F6.

In order to evaluate the molecular weight ranges of molecules contained in the different fractions, a mixture of standards (0.5% w/v) was injected on the SEC system. The chromatogram of the standards is depicted in the Figure 1.b. a) Laminarin (from about 3 to about 5kDa), b) Raffinose (594Da), c) Sucrose (343.3Da), d) Citrate salt (343.3Da), e) Mannitol (182.2Da) and f) Glycine (75.1Da). According to these results, the fraction F1 contained molecules with high molecular weights (higher than 4 kDa), the fraction F2 contained Laminarin (from about 3 to about 4kDa) which was eluted between F1 and F2 fractions.

An ultrafiltrate, obtained after ultrafiltration of the RM-2705 extract on an ultrafiltration system with a cut-off membrane of 1kDa, was injected on the SEC system. The chromatographic profile of the ultrafiltrate showed only the chromatographic peaks corresponding to those of the fractions F3, F4, F5 and F6 from the SEC fractionation of the RM-2705 extract. Thus, the fraction F3 contained molecules with molecular weights smaller than 1kDa to about 0.18kDa, Fraction F4 contained molecules with molecular weights smaller than about 0.2kDa such as Mannitol (182.2Da) and Fractions F5 and F6 contained salts and molecules very low molecular weights such as glycine (75Da). The NMR spectra of the different fractions confirmed the presence of sulfated polysaccharides (fucan polymers) in the first fraction F1 while the second fraction F2 contains laminarin (from about 3 to about 4kDa) and the fraction four F4 contains mannitol (182.2 Da). The last two fractions F5 and F6 contained very low molecular weight molecules and salts.

The different fractions obtained by SEC fractionation were tested for their plant growth stimulation activity on lettuces in comparison with the whole seaweed extract RM-3496. Before the injection on the Chromatography, the seaweed extract was filtered and this filtered extract was also tested on lettuce to check its efficacy. The results showed that bioactive molecules were found in the F3 fraction as illustrated in Figure 2. A significant activity was found in the F3 fraction which contained molecules ranging from about 0.15 kDa to about 1 kDa.

The combination of the different techniques used to desalt the RM-2705 extract provided information about the physicochemical properties of the bioactive molecule(s). In particular, it was determined that the bioactive molecules are polar and their molecular weights range from about 0.15 to about 1 kDa. Thus, this information excludes, from the growth-promoting bioactive polymers, fucan polymers which are the major sulfated polysaccharides in *Ascophyllum nodosum* acidic extract, laminarin (from about 3 to about 4kDa), a beta-1,3-glucan elicitor, and mannitol (182 Da), a polyol that can represent up to about 8-10% of the extract by dry weight.

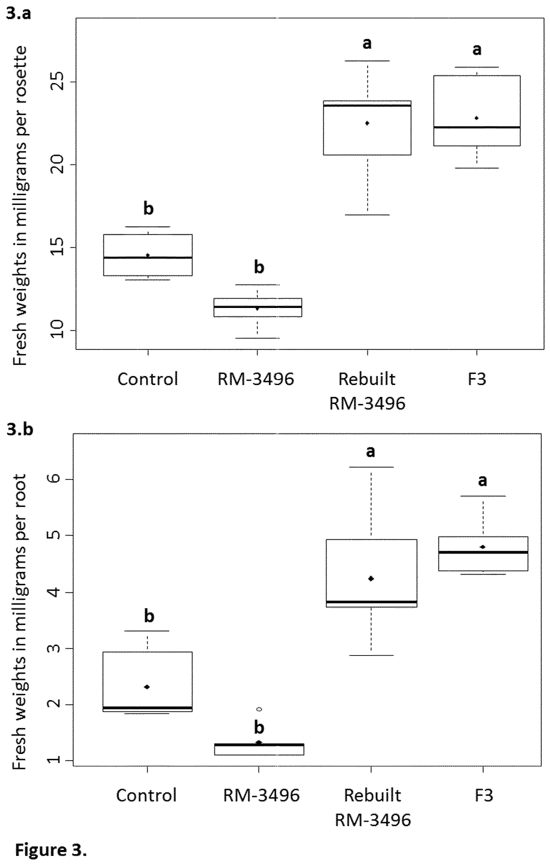

In order to confirm the bioactivity of the purified F3 fraction an *in-vitro* culture using the model plant *Arabidopsis thaliana* was developed and reproduced several times. These tests illustrated in Figure 3.a. confirmed the bioactivity of the F3 fraction whereas the presence of salts in the RM-3496 extract disturbed the growth of *Arabidopsis thaliana* in these culture conditions. However, the Rebuilt-RM-3496 corresponding to the reconstitution of the seaweed extract with the SEC fractions, displayed a growth promoting activity. Moreover, in this *In-vitro* bioassay, the fraction F3 and the Rebuilt-RM-3496 also appeared to enhance root growth as shown in Figure 3.b.

The fraction F3 displayed a strong growth-stimulating activity whereas fractions F1 and F2 were inactive. However, during the development of the latter bioassay, it was shown that in the presence of salt (100 mM NaCl), the fraction F3 was no longer active to stimulate growth, whereas F1 and F2 displayed similar effects, and that the whole RM-2705 extract confer salt tolerance. These results indicate that the RM-2705 extract contains different active compounds with different modes of action, including (1) the low molecular weight (LMW) fraction responsible for growth stimulation, and (2) fractions containing laminarin and fucans that confer stress tolerance (salt, as well as biotic, stress resistance).

Taken together, these results indicate that the fractionation of the RM-2705 extract can provide at least two types of products with distinct modes of actions and thus different applications using the same raw materials.

Since SEC is not transferrable at an industrial scale, it was desirable to also develop another method capable of producing fractionated products of seaweed extracts that can provide plant growth stimulant activity on a larger scale.

One alternative to SEC is ultrafiltration (UF), a fractionation process in which seaweed extract is filtered through ultrafiltration membranes with suitable molecular weight cutoffs (MWCO). The fraction of the present invention is obtainable by ultrafiltration of an extract of the algal species using a 1kDa MWCO membrane, and the fraction comprises bioactive molecules in the desired range of about 0.15 kDa to 1.0 kDa.

The present invention generally provides a method of purifying a biostimulant composition derived from a seaweed extract comprising a step of ultrafiltration using a semipermeable ultrafiltration membrane to separate the molecules of interest from the rest of the mixture according to their molecular weight, size and shape.

The ultrafiltration step may be carried out using ultrafiltration equipment in which a seaweed extract solution comprising between about 1% by wt. and about 15% by wt. dry matter, more preferably between about 2% by wt. and about 7% by weight dry matter, is subjected to ultrafiltration using a membrane with a suitable molecular weight cutoff (MWCO). In one embodiment, the ultrafiltration process involves tangential ultrafiltration.

The filtrate is collected for its biostimulant properties while recirculating the retentate (or concentrate), which is left apart for other applications at the end of the process. Although it is generally not necessary or required, if desired, a further purification of the retentate (or concentrate) can be achieved by the addition of fresh water at a rate corresponding to that at which water, together with molecules having a molecular weight less than or equal to 1 kDa is removed from the ultra-filtrate.

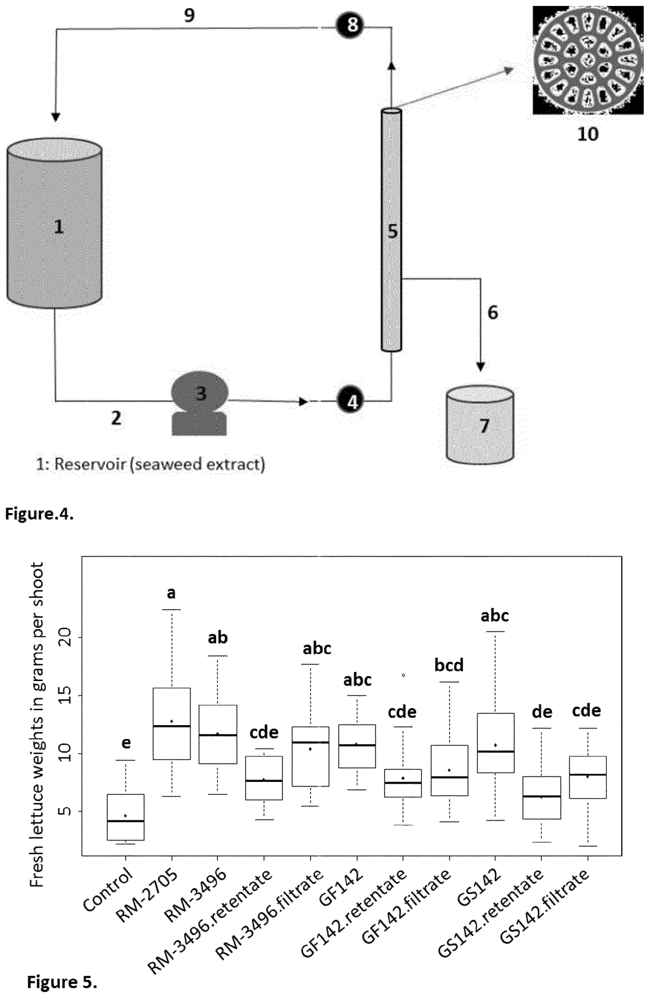

As seen in Figure 4, ultrafiltration can be carried by a process in which the solution reservoir (1) is charged with a batch of seaweed extract. The solution is circulated by line (2) and pump (3) into an inlet manifold (4) of an ultrafiltration unit (5). The ultrafiltration unit (5) comprises one or more cartridges arranged in parallel to provide the appropriate ultrafiltration membrane area. The ultra-filtrate then exits the ultrafiltration unit (5) via outlet line (6) and is collected in tank (7). The ultrafiltration concentrate exits the ultrafiltration unit (5) via outlet manifold (8) and is returned via line (9) to the solution reservoir (1).

The membrane contained in the ultrafiltration unit (5) may be polymeric or ceramic type membrane. In one embodiment, the membrane comprises a tubular ceramic membrane comprising a plurality of channels. For example, the membrane may contain between about 15 and about 50 channels, more preferably between about 19 and about 39 channels and may have a length between about 50 and about 150 cm. In other embodiments, spiral membranes and crossflow membranes may also be used in the practice of the invention. The membrane area is generally between about 0.20 and about 0.6 m², more preferably between about 0.30 and 0.40 m².

The retentate is rinsed several times to remove the major portion of molecules with a molecular weight smaller than the cutoff of the membrane. The ultra-filtrates contain molecules with a molecular weight smaller than that of the cutoff membrane. In the method of the invention, the cutoff is 1 kDa or less. The molecules that are contained in the ultra-filtrates display low molecular weights smaller than the MWCO, e.g., 1 kDa, and are commonly referred to as metabolites. The retentates contain molecules with molecular weights larger than the cutoff membranes, e.g., 1 kDa. The molecules that are contained in the retentate display high molecular weights (e.g., Laminarin from about 3 to about 4 kDa or Fucoidans higher than about 30 kDa and other brown algal high molecular weight biopolymers).

All algal species from the order of Fucales have been found to display a promising activity and can be subjected to the methods described herein. These algal species include, but are not limited to, species of the families of Fucaceae, Sargassaceae and Durveilleaceae. Other species from the Fucales and Laminariales orders include, but are not limited to Ascoseirales, Asterocladales, Desmarestiales, Dictyotales, Dictyotophycidae, Discosporangiales, Discosporangiophycidae, Ectocarpales, Fucales, Fucophycidae, Ishigeales, Ishigeophycidae, Laminariales, Nemodermatales, Onslowiales, Phaeophyceae ordo incertae sedis, Phaeosiphoniellales, Ralfsiales, Scytothamnales, Sphacelariales, Sporochnales, Stschapoviales, Syringodermatales, Tilopteridales, among others.

In addition, while the present invention is described and shown to demonstrate positive results on algal species from the order Fucales, the method is not limited to these algal species and can also be used to isolate and analyze filtrates of any algae or other species that may act as biostimulants to determine bioactivity of such filtrates.

As used in the Figures herein, the term "filtrate" refers to filtrate and ultra-filtrates obtained after one or more ultrafiltrations through the ultrafiltration unit.

As used in the Figures herein, the term "retentate" refers to retentate without flushing and retentates obtained after one or more flushings.

### Examples:

### Example 1:

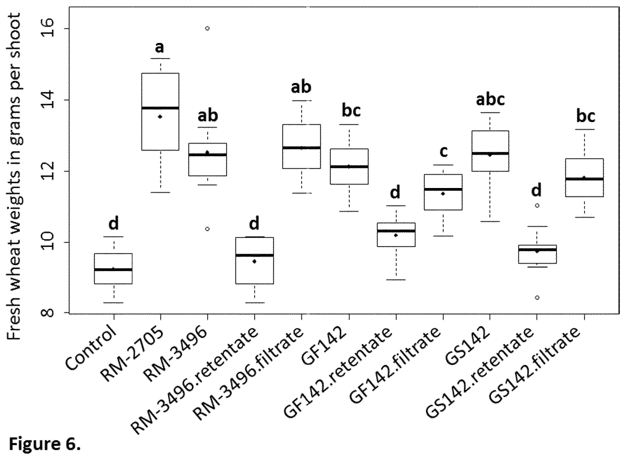

A RM-3496 extract was ultrafiltrated at the laboratory scale with a 1 kDa MWCO membrane and the retentate was rinsed three times with water. The ultra-filtrate, containing molecules with molecular weights smaller than 1 kDa, and the retentate, containing molecules with molecular weights larger than 1 kDa were tested on lettuces and wheat. Thus, the different filtrates and retentates were applied on lettuces and wheats. The GF142 and GS142 extracts (available from Laboratoires Goëmar) were manufactured with the same process from *Fucus vesiculosus* and *Sargassum natans* respectively. The results are shown in Figures 5 and 6, which depicts boxplots showing the fresh shoot weights of the control plants, plants treated with four different seaweed extracts (RM-2705, RM-3496, GF142 and GS142), the plants treated with high molecular weight molecules correspond to retentates named: RM-3496.retentate, GF142.retentate and GS142.retentate, and the plants treated with low molecular weight molecules correspond to filtrates named: RM-3496.filtrate, GF142.filtrate and GS142.filtrate. These results confirm the efficacy of seaweed extracts and show a growth promoting activity in the filtrates, where the retentates appear inefficient in promoting plant growth.

### Example 2:

Ten liters of an aqueous extract from *Ascophyllum nodosum* (pH 2.76) were placed in a solution reservoir of a pilot scale ultrafiltration unit fitted with a 58 cm long, tubular (diameter: 25 mm, 23 channels, cut-off 1 kDa) ceramic ultrafiltration membrane (supplied by Tami Industries). The solution was pumped through the ultrafiltration tube with complete recirculation of the concentrate back to the reservoir. Six liters of filtrate were collected and identified as F1. The retentate (4 L) was rinsed twice with 5 liters of water to produce 2 filtrates (F2 = 5 L; F3 = 5 L). The different filtrates (F1, F2, F3) were further evaluated for their biostimulant properties.

### Example 3:

Five liters of an aqueous extract from *Fucus vesiculosus* (pH 2.42) were placed in a solution reservoir of a pilot scale ultrafiltration unit, fitted with a 58 cm long, tubular (diameter: 25 mm, 23 channels, cut-off 1 kDa) ceramic ultrafiltration membrane (supplied by Tami Industries). The solution was pumped through the ultrafiltration tube with complete recirculation of the concentrate back to the reservoir. The filtrate was collected and identified as F1. The retentate (2.5 L) was rinsed once with 2.5 L of water to produce 2.5 liters of filtrate (F2 = 2.5 L). The different filtrates (F1, F2) were further evaluated for their biostimulant properties.

### Example 4:

Five liters of an aqueous extract from *Sargassum natans* (pH 2.92) were placed in a solution reservoir of a pilot scale ultrafiltration unit, fitted with a 58 cm long, tubular (diameter: 25 mm, 23 channels, cut-off 1 kDa) ceramic ultrafiltration membrane (supplied by Tami Industries). The solution was pumped through the ultrafiltration tube with complete recirculation of the concentrate back to the reservoir. The filtrate was collected and identified as F1. The retentate (2.5 L) was rinsed once with 2.5 L of water to produce 1 liter of filtrate (F2 = 2.5 L). The different filtrates (F1, F2) were further evaluated for their biostimulant properties.

### Example 5:

RM-3496, manufactured by Laboratoires Goëmar from *Ascophyllum nodosum* extract and two other seaweed extracts (GF142 and GS142, manufactured by Laboratoires Goëmar from *Fucus vesiculosus* and *Sargasssum natans* respectively) were subjected to ultrafiltration and evaluated for their biostimulant properties.

These three fucoid extracts were ultrafiltrated on a ceramic membrane (available from TAMI Industries) having a suitable MWCO (i.e., 1 kDa). Ten liters of RM-3496 were ultrafiltrated and five liters of the ultrafiltrate were collected and constituted the filtrate 1 used in additional experiments on lettuce and wheat. The retentate (5L) was rinsed twice with 5 liters of water, while GF142 and GS142 retentates (2.5L) were rinsed only once with 2.5 liters of water. The dry weights of the filtrates, ultra-filtrates and retentates were measured. According to the fractionation process of the RM-3496 extract, the total dry weights of the filtrates (containing molecules with molecular weights smaller than 1 kDa) was about 80% of the RM-3496 extract and the retentate was about 20% of the RM-3496 extract.

The details of the plant growth experiments are described below.

The treatments were performed with different Goëmar's extracts (RM-2705, RM-3496, GF142 and GS142) and a dilution factor of 250 (equivalent to 4 milliliters of liquid extract per liter of nutritive solution) was used for all experiments. The different fractions resulting from the SEC fractionation and from the Ultrafiltration fractionation were applied on plants according to their purification yields which were calculated with dry weights. Several independent biological repetitions were performed with the different fractions with n plants by treatments

The lettuce growth experiments were performed with seeds of lettuces *Lactuca sativa* ecotypes Fabietto or Janero (available from Voltz, Colmar, France). Lettuces were grown in a growth-chamber, on a rotary table to obtain plant phenotype as homogeneous as possible for any condition of treatment. Plants were grown under high pressure iodide-sodium lamps with a photosynthetically active radiation of 150 ± 10 µmol of photons.m-2.s-1, a thermo-period of 20/18°C (day/night) and a long-day photoperiod of 16h light. In order to control the nutrient inputs to plants and to facilitate the roots gathering, seeds of lettuces were grown in sand pots. Plants were watered three times per week with a commercial nutritive solution (available from Puteaux, Les Clayes-sous-Bois, France) having nitrogen, phosphate, and potassium concentrations in a ratio of N/P/K 20:20:20 (1 g/L)

Lettuces were treated twice (once/week at days 21^{st} and 28^{th}) with the different seaweed extracts and fractions were added to the nutritive solution and the bases of the pots were immersed in nutritive solution until total absorption was observed.

The plants were harvested 16 days after the first treatment, and the shoots and roots were gathered separately. Three independent biological repetitions were performed with the different seaweed extracts and fractions. Twelve lettuces (n=12) were used by treatments for the SEC fractionation experiments while eighteen lettuces (n=18) were used by treatments for the ultrafiltration experiments.

Seeds of *Arabidopsis thaliana* ecotype Columbia (Col-0 obtained from the ABRC seed stock center) were grown in in-vitro cultures. Seeds were first surface-sterilized and were sown in squared Petri dishes containing Half-strength Murashige and Skoog (MS) basal medium supplemented with 1% (w/v) of sucrose (30mM) and 0.6% (w/v) of PhytagelTM. Petri dishes were grown under a cool fluorescent light with an intensity of 225 ±10 µmol photons.m-2.s-1, with a long-day photoperiod of 16h light at 21°C ± 0.5°C. The location of Petri plates under the neon lamps were changed every day and this all along experiment to randomize the experiment.

Plantlets with uniform growth were selected and transferred 6 days after germination on treatment media. For each condition, 6 Petri dishes containing 6 plantlets each were prepared

The plants were gathered 9 days after the transfer on treatment media. Four independent bioassays were performed and six replicates (n=6) were used by treatments for the SEC fractionation experiments.

The wheat growth experiments were performed with seeds of winter wheat (*Triticum aestivum* L.) variety Altigo (available from Limagrain, Saint-Beauzire, France). Wheats were grown in a growth chamber on a rotary table to obtain for each condition plant phenotype as homogenous as possible. In order to control the nutrient inputs to the plants, seeds of wheat were grown in vermiculite pots. The plants were grown in the growth-chamber under high pressure iodide-sodium lamps with a photosynthetically active radiation of 150+/- 10 µmol of photons.m⁻².s⁻¹ and a thermo-period of 22/18°C with a long day photoperiod of 16 hours. Ten days after sowing, homogeneous plants were distributed in different trays; 6 plants per tray and two trays per condition. The plants were watered three times per week with the same commercial nutritive solution used for lettuce experiments.

Two weeks after sowing, the wheats were treated fivefold (every 2 or 3 days) with the different fractions and extracts and were harvested 13 days after the first treatment. The efficacy of the different fractions was assessed by comparison of fresh shoot weights. Three independent biological repetitions were performed with the different seaweed extracts and fractions. Twelve wheats (n=12) were used by treatments for the ultrafiltration experiments.

In the present invention, the efficacy of the different fractions and extracts on plant growth stimulation was evaluated by a statistical approach. Indeed, for each bioassay shown, the normality of the data was first checked with Shapiro-Wilk normality tests, with the Q-Q plots and with the histograms of density. The Homoscedasticity of these data was also checked with the Barlett's test, prior to performed parametric tests on these data. Several bioassays (three to four independent repetitions in time) were carried out to assess the different treatments on plant growth stimulation with a number N of plants by Treatment. A parametric two-way analysis of variance (two-way Anova) was then performed on the data to determine if there was a significant difference (with an alpha error of 5%) between the means of the different treatments for each bioassay and between the means of each treatment for the different bioassays carried out. According to the Anova results, a parametric post-hoc HSD Tukey's test or multiple pairwise comparison was performed on the data to range and define what means were significantly different from each other. Tukey's test results are shown on the boxplots with bold letters. The means of treatments which are significantly different from each other display different bold letters. These means are depicted on each boxplot by a dot.

The compounds described herein can be used on various crops including, for example soybeans, corn, cereals (i.e., wheat, barley, rye, and oats), rapeseed, canola, sunflower, sugar beet, potatoes, dry pulses (i.e., lentils, dry beans, etc.), sugarcane, fruiting vegetables, including tomatoes, eggplant, peppers, cucurbits, etc., bulb vegetables, including onions and leeks, head and leafy vegetables, including lettuce, spinach and celery, brassicas, stone fruits, pome fruits, citrus, coffee, cocoa, nut trees, berries, grapes (tables and vines), among others.

## Claims

1. A fraction isolated from an algal species, comprising one or more bioactive molecules having a molecular weight in the range of 0.15 kDa to 1.0 kDa, wherein:
the fraction is obtainable by ultrafiltration of an extract of the algal species using a 1 kDa MWCO membrane, and the one or more bioactive molecules do not comprise a sulfated polysaccharide or laminarin;
the algal species is a brown algal species selected from the group comprising *Ascophyllum nodosum, Fucus vesiculosus, Sargassum* sp., and combinations thereof; and
the fraction is for improving plant growth.

2. A method of improving plant growth, the method comprising the step of applying a composition comprising the fraction of claim 1 to at least one of soil, a plant, or a seed.

3. The method of claim 2, wherein improving plant growth includes at least one of the following: promoting seed germination, stimulating root development, prolonging a vegetative period, increasing a period of production, or increasing a period of harvest.

4. A method of isolating and purifying bioactive compounds in an extract obtained from seaweed, the method comprising the steps of:
a) circulating the extract through an ultrafiltration membrane having a suitable molecular weight cutoff of 1 kDa or less;
b) collecting filtrate from the extract to obtain a first filtrate fraction and a retentate; and
c) rinsing the retentate to obtain one or more additional filtrate fractions.

5. The method according to claim 4, wherein the extract is produced from a brown algal species.

6. The method according to claim 4 or claim 5, wherein the extract is obtained from *Ascophyllum nodosum, Fucus vesiculosus,* or *Sargassum* sp. algae.

7. The method according to any of claims 4 to 6, wherein the retentate comprises active molecules selected from the group consisting of sulfated polysaccharides and laminarin, and
wherein the active molecules are capable of alleviating abiotic stress in crops.

8. The method according to any of claims 4 to 7, wherein the first filtrate comprises bioactive molecules having a molecular weight in the range of 0.15 kDa to 1.0 kDa.

9. Use of a fraction isolated from an algal species to improve plant growth, the fraction comprising one or more bioactive molecules having a molecular weight in the range of 0.15 kDa to 1.0 kDa, wherein:
the fraction is obtainable by ultrafiltration of an extract of the algal species using a 1 kDa MWCO membrane, and the one or more bioactive molecules do not comprise a sulfated polysaccharide or laminarin; and
the algal species is a brown algal species selected from the group comprising *Ascophyllum nodosum, Fucus vesiculosus, Sargassum* sp., and combinations thereof.

## Patentansprüche

1. Aus einer Algenart isolierte Fraktion, die ein oder mehrere bioaktive Moleküle mit einem Molekulargewicht im Bereich von 0,15 kDa bis 1,0 kDa umfasst, wobei:
die Fraktion durch Ultrafiltration eines Extrakts der Algenart unter Verwendung einer Membran mit einem Molekulargewichtsgrenzwert (MWCO) von 1 kDa erhältlich ist, und das eine oder die mehrere bioaktiven Moleküle kein sulfatiertes Polysaccharid oder Laminarin umfassen;
die Algenart eine Braunalgenart ist, ausgewählt aus der Gruppe, die *Ascophyllum nodosum, Fucus vesiculosus, Sargassum sp.* und Kombinationen davon umfasst; und
die Fraktion zur Verbesserung des Pflanzenwachstums dient.

2. Verfahren zur Förderung des Pflanzenwachstums, wobei das Verfahren den Schritt des Aufbringens einer Zusammensetzung, die die Fraktion nach Anspruch 1 enthält, auf mindestens eines von Boden, einer Pflanze oder einem Samen umfasst.

3. Verfahren nach Anspruch 2, wobei die Förderung des Pflanzenwachstums mindestens eines der Folgenden enthält: Fördern der Samenkeimung, Stimulieren der Wurzelentwicklung, Verlängern einer Vegetationsperiode, Verlängern einer Produktionsperiode oder Verlängern einer Ernteperiode.

4. Verfahren zur Isolierung und Reinigung bioaktiver Verbindungen in einem aus Meeresalgen gewonnenen Extrakt, wobei das Verfahren folgende Schritte umfasst:
a) Zirkulieren des Extrakts durch eine Ultrafiltrationsmembran mit einer geeigneten Molekulargewichtsgrenze von 1 kDa oder weniger;
b) Sammeln des Filtrats aus dem Extrakt, um eine erste Filtratfraktion und ein Retentat zu erhalten; und
c) Spülen des Retentats, um eine oder mehrere zusätzliche Filtratfraktionen zu erhalten.

5. Verfahren nach Anspruch 4, wobei der Extrakt aus einer Braunalgenart hergestellt wird.

6. Verfahren nach Anspruch 4 oder Anspruch 5, wobei der Extrakt aus *Ascophyllum nodosum, Fucus vesiculosus* oder *Sargassum sp.* gewonnen wird.

7. Verfahren nach einem der Ansprüche 4 bis 6, wobei das Retentat aktive Moleküle umfasst, die aus der Gruppe ausgewählt sind, die aus sulfatierten Polysacchariden und Laminarin besteht, und
wobei die aktiven Moleküle in der Lage sind, abiotischen Stress bei Kulturpflanzen zu lindern.

8. Verfahren nach einem der Ansprüche 4 bis 7, wobei das erste Filtrat bioaktive Moleküle mit einem Molekulargewicht im Bereich von 0,15 kDa bis 1,0 kDa umfasst.

9. Verwendung einer aus einer Algenart isolierten Fraktion zur Verbesserung des Pflanzenwachstums, wobei die Fraktion ein oder mehrere bioaktive Moleküle mit einem Molekulargewicht im Bereich von 0,15 kDa bis 1,0 kDa umfasst, wobei:
die Fraktion durch Ultrafiltration eines Extrakts der Algenart unter Verwendung einer Membran mit einem Molekulargewichtsgrenzwert (MWCO) von 1 kDa erhältlich ist und das eine oder die mehreren bioaktiven Moleküle kein sulfatiertes Polysaccharid oder Laminarin umfassen; und
die Algenart eine Braunalgenart ist, ausgewählt aus der Gruppe, die *Ascophyllum nodosum, Fucus vesiculosus, Sargassum sp.* und Kombinationen davon umfasst.

## Revendications

1. Fraction isolée d'une espèce algale, comprenant une ou plusieurs molécules bioactives ayant un poids moléculaire dans la plage de 0,15 kDa à 1,0 kDa, dans laquelle :
la fraction peut être obtenue par ultrafiltration d'un extrait de l'espèce algale en utilisant une membrane MWCO de 1 kDa, et l'une ou les plusieurs molécules bioactives ne comprennent pas de polysaccharide ou de laminarine sulfaté(e) ;
l'espèce algale est une espèce algale brune sélectionnée parmi le groupe comprenant *Ascophyllum nodosum, Fucus vesiculosus, Sargassum sp.,* et combinations de celles-ci ; et
la fraction est destinée à améliorer la croissance des plantes.

2. Procédé pour améliorer la croissance des plantes, le procédé comprenant l'étape consistant à appliquer une composition comprenant la fraction de la revendication 1 sur au moins un composant parmi un sol, une plante, ou une semence.

3. Procédé de la revendication 2, dans lequel le fait d'améliorer la croissance des plantes inclut au moins un des faits suivant : favoriser la germination de semences, stimuler le développement des racines, prolonger une période végétative, augmenter une période de production, ou augmenter une période de récolte.

4. Procédé pour isoler et purifier des composés bioactifs dans un extrait obtenu d'algue marine, le procédé comprenant les étapes suivantes :
a) faire circuler l'extrait à travers une membrane d'ultrafiltration ayant une limite de poids moléculaire appropriée de 1 kDa ou moins ;
b) collecter un filtrat de l'extrait pour obtenir une première fraction de filtrat et un rétentat ; et
c) rincer le rétentat pour obtenir une ou plusieurs fractions de filtrat supplémentaires.

5. Procédé selon la revendication 4, dans lequel l'extrait est produit d'une espèce algale brune.

6. Procédé selon la revendication 4 ou la revendication 5, dans lequel l'extrait est obtenu d'algues *Ascophyllum nodosum, Fucus vesiculosus,* ou *Sargassum sp.*

7. Procédé selon de quelconques des revendications 4 à 6, dans lequel le rétentat comprend des molécules actives sélectionnées parmi le groupe constitué de polysaccharide et de laminarine sulfaté(e), et
dans lequel les molécules actives sont capables de réduire l'agression abiotique dans des cultures.

8. Procédé selon de quelconques des revendications 4 à 7, dans lequel le premier filtrat comprend des molécules bioactives ayant un poids moléculaire dans la plage de 0,15 kDa à 1,0 kDa.

9. Utilisation d'une fraction isolée d'une espèce algale pour améliorer la croissance des plantes, la fraction comprenant une ou plusieurs molécules bioactives ayant un poids moléculaire dans la plage de 0,15 kDa à 1,0 kDa, dans lequel :
la fraction peut être obtenue par ultrafiltration d'un extrait de l'espèce algale en utilisant une membrane MWCO de 1 kDa, et l'une ou les plusieurs molécules bioactives ne comprennent pas de polysaccharide ou de laminarine sulfaté(e) ; et
l'espèce algale est une espèce algale brune sélectionnée parmi le groupe comprenant *Ascophyllum nodosum, Fucus vesiculosus, Sargassum sp.,* et combinations de celles-ci.
